# EUROPEAN PATENT APPLICATION

(11) **EP 3 213 712 A1**
(43) Date of publication of application: **06.09.2017**
(21) Application number: 17275026.7
(22) Date of filing: 28.02.2017
(51) Int. Cl.: A61B 34/00, A61B 18/00

(54) **MAGNETIC GUIDANCE SYSTEM PARTICULARLY FOR NEUROLOGICAL APPLICATIONS**

(30) Priority: 02.03.2016 GB 201603636
(71) Applicant: Cook Medical Technologies LLC, Bloomington, IN 47404 (US)
(72) Inventor: Hansen, Palle Munk, 4632 Bjaeverskov (DK); Andersen, Torben Peter, 2630 Taastrup (DK)
(74) Representative: Williams Powell

(57) **Abstract**

A magnetic guidance system (20) for guiding a guidewire (70) or other elongate medical device includes a magnet unit (20) disposed in one implementation adjacent a head end of a patient table (30). The magnet unit is for example disposed on a magnet support which enables the magnet unit (20) to move in a periodic motion substantially around a single plane. The preferred motion is a rotary motion about a patient's head, which creates a repetitive repulsive force at the distal end of the medical device, causing the distal end of the medical device to move in different directions within the patient's vasculature, useful in assisting the guidance of the medical device through tortuous vessel structures. The repetitive alternating medical field makes guidance of a medical device within a patient's vasculature a relatively simple task.

## Description

### Technical Field

The present invention relates to a magnetic guidance system for guiding a medical device within a patient, in the preferred embodiment for guiding a guide wire, catheter or other endoluminal device through the vasculature of a patient, particularly for neurological applications.

### Background of the Invention

Endoluminal medical procedures are commonly practiced for a variety of medical procedures, such as for vessel or valve repair, angioplasty procedures, treatment of aneurysms, implantation of medical devices, localised delivery of medicinal products and so on. Most of these procedures involve the insertion of an elongate element from a remote percutaneous entry point of the patient, into a vessel of the patient and then the feeding of the device through the patient's vasculature to the treatment point. A variety of techniques are known, a commonly used procedure being the well-known Seldinger technique.

The process of feeding a medical assembly through a patient's vasculature is a specialised technique but commonly practiced. Where insertion must be carried out through tortuous or delicate vessels, the procedure can be complicated. Difficulties arise particularly in neurological applications, where the cerebral vessels are very delicate, very tortuous and prone to vessel spasm. It can often take several hours to position a guide wire for an endoluminal neurological procedure. In such a case, the physician must carefully guide the distal end of the guide wire through the vessels, often removing the guide wire to adjust its curvature to pass through a particular vessel curve or bifurcation. Despite these time and technical difficulties, an endoluminal procedure is still much more preferable over open surgery.

Attempts have been made to provide guidance systems for guiding a guide wire through a patient's vasculature. Examples can be found in US-2007/0016006, US-2006/0142632, US-2004/0019447, US-2003/0040737, US-6,296,604, US-2013/0131503. In general terms, these documents disclose magnetic guidance systems for guiding a guide wire having a magnet at its distal end. The systems use a framework of permanent or electromagnets arranged around the patient, at least some of which are movable relative to the patient during the procedure. The magnets provide an attractive force on the distal tip of the guide wire which can be used to direct the guide wire through the patient's vasculature. The systems generally provide a user input, which may for instance be in the form of a joystick or the like. The user input is used to move the location of the guiding magnets and the magnetic force applied to the distal end of the guide wire through the patient's vessels. Once the guide wire has been positioned as required, the subsequent medical procedure can be conducted in conventional manner.

There are a number of shortcomings with these systems which it is believed limit their practical application. The systems are large and complex; they envelop a patient, restricting access to the patient and leading to patient anxiety; and also require training and additional manipulation by the physician.

### Summary of the Present Invention

The present invention seeks to provide an improved magnetic guidance system for guiding a medical device within a patient, in the preferred embodiment for guiding a guide wire, catheter or other endoluminal device through the vasculature of a patient, particularly for neurological applications.

According to an aspect of the present invention ,there is provided a medical magnetic guidance system as in claim 1.

According to an aspect of the present invention there is provided a medical magnetic guidance system for guiding a medical device deployed in a patient, the system including:
a patient support;
a magnet assembly disposed adjacent the patient support, the magnet assembly including a movable magnet support and one or more magnets attached to the support, the magnet support being mounted on a guide element having a single plane;
a drive mechanism coupled to the magnet support to move the magnet support and the one or more magnets attached thereto in the guide element in said single plane.

In a preferred embodiment, the medical magnetic guidance system is designed for guiding a medical neurological device deployed in a patient, the system including: a patient support including a patient head zone and a patient body zone; a magnet assembly disposed adjacent the patient head zone, the magnet assembly including a movable magnet support and one or more magnets attached to the support; a drive mechanism coupled to the magnet support and operable to move the magnet support and the one or more magnets attached thereto in a single plane relative to the patient head zone of the patient support.

This aspect of the present invention provides a system in which the magnets are maintained in a single plane relative to a patient's body. This provides the advantage of keeping the patient's body accessible to the physician, for imaging purposes, and also avoids enveloping the patient in machinery, which can be stressful and not conducive to smooth operating conditions. This is particularly beneficial also for neurological applications.

In the preferred embodiment, the drive mechanism is operable to move the magnet support in a rotary, reciprocating or vibratory motion in said single plane. As is explained below, this provides an effective mechanism for controlling the movement of a medical device within the patient and which can also avoid complex control inputs by the physician or other medical staff.

The drive mechanism is preferably operable to move the magnet support in a repetitive periodic motion in said single plane. As is set out below in the specific description of the preferred embodiments, this feature enables the physician to move the endoluminal medical device under the influence of the periodic, and repetitive, moving magnetic field without the need for any other control input.

The patient support may include a patient head zone and a patient body zone.

The single plane may be lateral, above or below the patient support. In a preferred embodiment, the single plane may be lateral, above or below the patient head zone of the patient support. Most preferably, the single plane is at or adjacent a crown end of the patient head zone. This is closest to the cerebral vessels, requiring therefore a smaller magnetic field, and can in practice allow the apparatus to be visually hidden from the patient.

According to another aspect of the present invention, there is provided a medical magnetic guidance system for guiding a medical device deployed in a patient, the system including:
a patient support;
a magnet assembly disposed adjacent the patient support, the magnet assembly including a movable magnet support, and one or more magnets attached to the support; the movable magnet support being mounted on a guide element;
a drive mechanism coupled to the magnet support to move the magnet support and the one or more magnets attached thereto in the guide element in a repetitive periodic motion.

As with the first aspect disclosed above, the magnet assembly may include a rotary coupling to magnet support, the drive mechanism being operatively connected to the rotary coupling so as to move the magnet support in a rotary motion. The magnet assembly may include a linear coupling to magnet support, the drive mechanism being operatively connected to the linear coupling so as to move the magnet support in a reciprocating motion.

In an embodiment, the magnet assembly includes a vibratory coupling to magnet support, the drive mechanism being operatively connected to the vibratory coupling so as to move the magnet support in a reciprocating vibratory motion.

As with the previous aspect, and all other aspects, the apparatus is preferably for neurological applications, in which case the patient support preferably includes a patient head zone and a patient body zone and said magnet assembly is disposed adjacent the patient head zone. The medical device is preferably a neurological device.

According to another aspect of the present invention, there is provided a medical magnetic guidance system for guiding a medical device deployed in a patient, the system including:
a patient support;
a magnet assembly disposed adjacent the patient support, the magnet assembly including a movable magnet support and one or more magnets attached to the support, the magnet support being mounted on a rotatable axle;
a drive mechanism coupled to the axle of the magnet support and operable to rotate the magnet support and the one or more magnets attached thereto about the axle.

The magnet support is preferably rotatable in a single plane.

All aspects of the present invention may include on or more of the following features.

The patient support may lie in a plane and the at least one magnet is disposed substantially perpendicular to the plane of the patient support. For this purpose, the at least one magnet may be disposed at or adjacent a top of head position of the patient support, in some embodiments in a plane tangential to a top of head position of the patient support.

Advantageously, the one or more magnets are positioned off-centre relative to the patient head zone of the patient support.

The magnet assembly may be positioned substantially in contact with the patient support.

The drive mechanism may be operable to move the magnet support in a periodic motion of around 1 rpm or less or 1/50 Hz or less.

In some embodiments, the one or more magnets may move within a range of 5 millimetres to 100 millimetres.

The drive mechanism is preferably operable to move the magnet support in a periodic motion 1 rpm or less, or 1/50 or 1/60 Hertz or less for non-rotary reciprocating motion.

The one or more magnets preferably move within a range of a few millimetres up to around 20 centimetres. As described in detail below, the magnet may also be angled towards the patient during the procedure.

In some embodiments, all magnets of the medical magnetic guidance system are attached to the magnet support.

The systems preferably include an elongate medical device, the medical device including a distal end magnetised distal tip having a given magnetic polarisation at the distal end, the one or more magnets having a front surface, which front surface has the same magnetic polarisation as that of the distal end of the medical device such that the one or more magnets exert a magnetic repulsive force on the distal end of the medical device.

As is explained below, the inventors have discovered that using repulsive magnetic forces can in practice provide much simpler guidance, in particular by reducing the movement required of the magnets to produce movement of the tip of the elongate medical device. This is a significant departure from earlier published systems.

The medical device may be a guide wire, a catheter or any other endoluminal medical device, including for instance another component of an introducer assembly, a probe or other medical tool.

Preferably, the magnet assembly is adjustable in yaw or pitch. Such adjustment can usefully be effected before the magnet(s) are put into motion, although in some embodiments the yaw and/or pitch of the magnet(s) could be adjusted while they are being operated, as a fine adjustment particularly for very tortuous vessels or at difficult vessel bifurcations.

Other features, aspects and advantages of the teachings herein will become apparent from the specific description which follows.

### Brief Description of the Drawings

Embodiments of the present invention are described below, by way of example only, with reference to the accompanying drawings, in which:
Figures 1 A and 1 B show in schematic form a preferred arrangement of magnets for a magnetic guidance system for neurological applications;
Figures 2A and 2B show in schematic form the arrangement of Figures 1 A and 1 B with an imaging device disposed about a patient's head area;
Figure 3 is a schematic diagram of an example of a magnetic guide wire suitable for use in the guidance system disclosed herein;
Figures 4A to 4C show the movement of the guide wire of Figure 3 under the influence of a repulsive magnetic force;
Figures 5A and 5B show how the guide wire of Figure 3 can be moved within a vessel labyrinth by the influence of a repulsive magnetic force;
Figures 6 and 7 show in schematic form the arrangement of magnets and magnet support for a neurological system, which depict also the preferred directions and orientations of adjustment of the magnet positions;
Figures 8 to 11 show in schematic for an embodiment of neurological guidance apparatus fitted to a patient support table;
Figures 12 and 13 show different views of a prototype magnet drive unit for a neurological magnetic guidance system;
Figures 14 to 18 show how a guide wire can be guided through tortuous vessels and around tight angled bends by the system disclosed herein; and

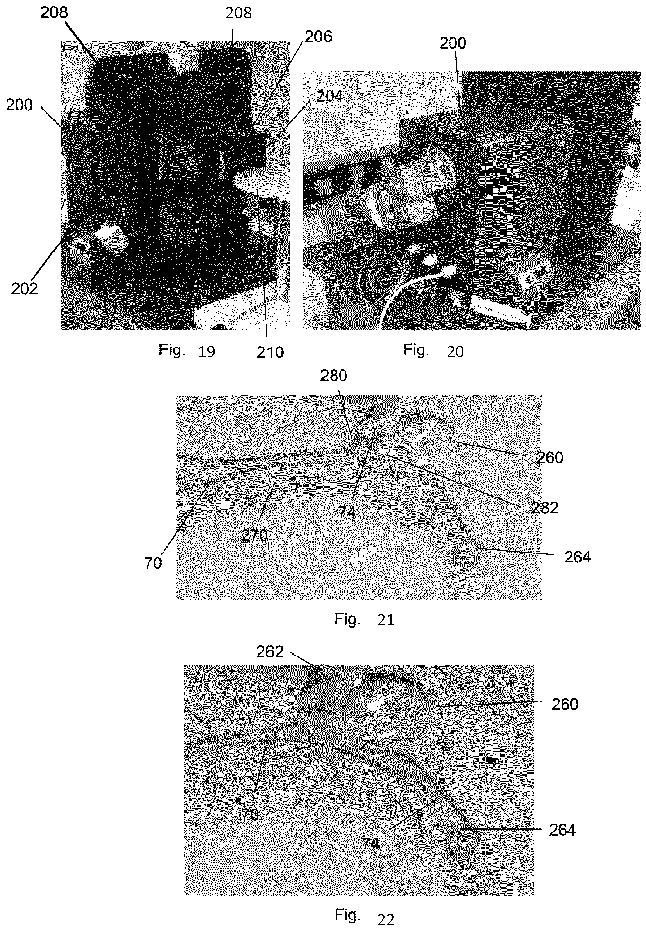

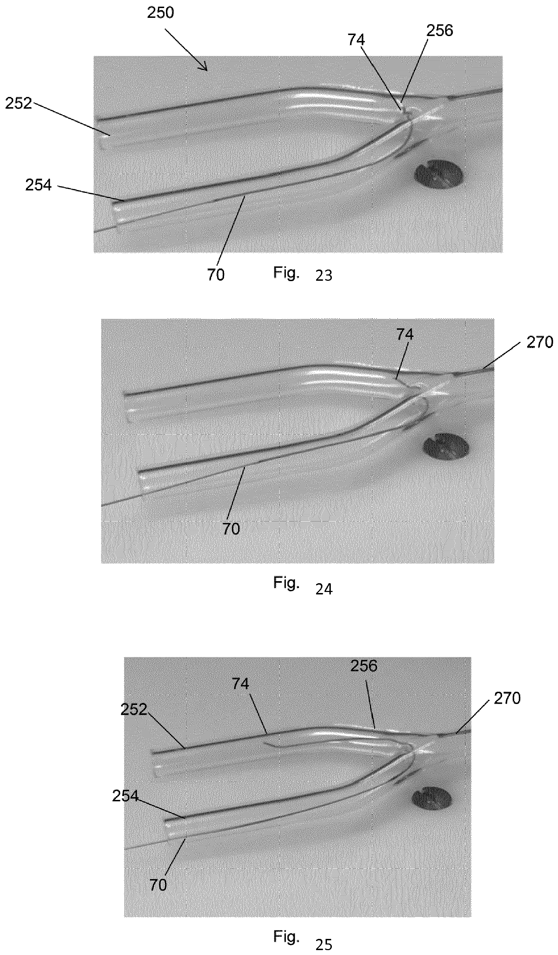

Figures 19 to 25 are photographs corresponding, respectively, to Figures 12 to 18.

### Description of the Preferred Embodiments

It is to be appreciated that the drawings show various features of the embodiments of the system taught herein in schematic form only. Moreover, not all of the features which would be included in such a device are shown in the drawings, which are intended to depict only the primary features of the disclosed system.

The preferred embodiments described below are directed to a neurological guidance system but it is to be understood that the teachings are not limited to neurological applications only. Other systems could readily be designed for other parts of the body of a patient. The principles and basic components of the apparatus can remain the same.

Moreover, the embodiments described below use a guide wire having a magnetic tip. The teachings herein, however, are not restricted to guiding a guide wire and are equally applicable to other endoluminal medical devices. One example is a catheter having a magnetic tip. A catheter of such a nature could be deployed through the vasculature of a patient without needing to use a guide wire.

The embodiments described herein may use permanent magnets or electromagnets. Both have relative advantages which are well-known to the person skilled art and therefore will not be discussed herein in detail.

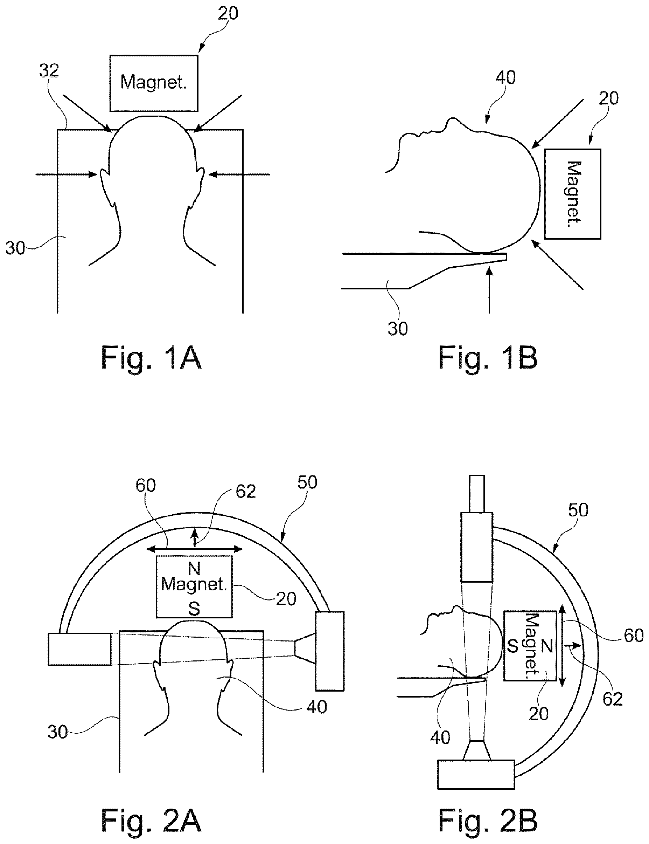

Referring first to Figures 1 A and 1 B, these show in schematic form and in general terms the arrangement of a magnetic unit for a guidance system of the type disclosed herein, designed for neurological applications. The arrangement depicted in Figure 1 A includes a patient support 30 which may be a table or similar support surface preferably of a nature on which a patient 40 can lie. Disposed at a head end 32 of the table 30 is a magnet unit 20. As will be apparent from Figures 1 A and 1 B, the magnet unit 20 is preferably located at the patient's crown when the patient is made to lie on the table 30. The magnetic unit 20 is preferably compact, such that a patient's head remains accessible to the physician as well as to other apparatus. The arrows shown in Figures 1 A and 1 B depict examples of access points to the patient's head for such purposes.

In this location, the magnet unit 20 is not visible to the patient during use and it can also be hidden, for example in a casing or behind a partition screen or curtain.

Referring now to Figures 2A and 2B, the guidance system of Figures 1 A and 1 B is shown together with an imaging device 50, which may be a magnetic resonance imaging unit or any other suitable imaging apparatus. As will be apparent from Figures 2A and 2B, the imaging device 50 can be positioned in a variety of orientations relative to the patient's head 40 and at each beyond the location of the magnet unit 20, thereby not to be obscured by the unit 20. As a result, a clean line of sight of the imaging equipment 50 can be obtained. Figures 2A and 2B also depict arrows 60, 62, indicative of possible directions of movement of the magnet unit 20 during operation of the system. As can be seen, the preferred directions of movement lie outside the line of sight of the imaging equipment 50 and can also remain out of sight of the patient's view, which can substantially reduce patient anxiety. The magnet unit 20 is typically fitted to a guide element so as to be movable as shown. All embodiments disclosed herein will include a guide element of a suitable form, whether as rails, tracks, bearings, a rotatable shaft of other known arrangement. The guide element will be immediately apparent to the skilled person in the examples which follow.

The magnet unit 20 depicted in schematic form of Figures 1 A to 2B is a significantly simpler arrangement compared to that of prior art systems of the types indicated above, and in the preferred embodiments achieve this by using a repulsive magnetic system as explained below.

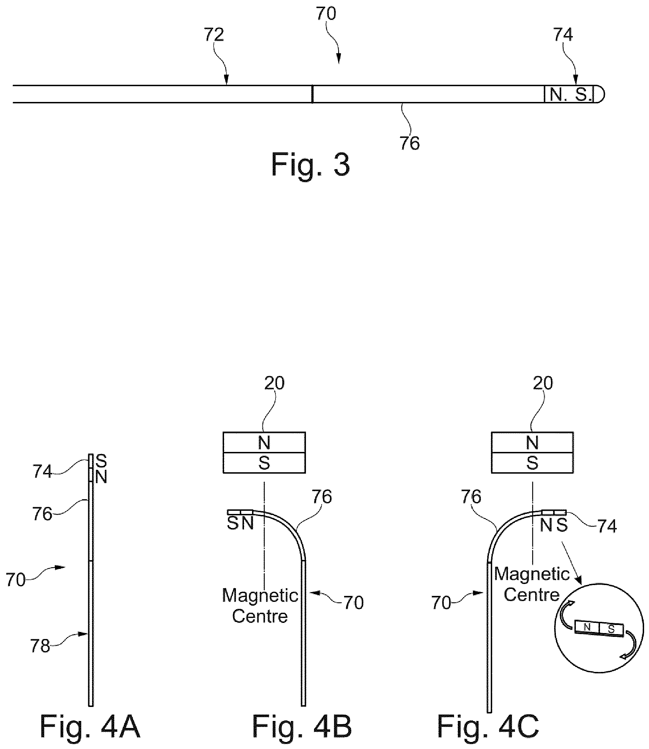

With reference now to Figure 3, this shows in very schematic form an example of a guidewire 70 of a type suitable for use in the system disclosed herein. The guidewire 70 includes an elongate body portion 72 which may have a conventional structure, for instance with a relatively flexible distal end 76 and a relatively less flexible proximal end 70, such that the distal end 76 has good trackability to pass through tortuous vasculature and the proximal end 72 has good pushability to help in pushing the guidewire 70 through a patient's vessel system.

The guidewire 70 differs from conventional guidewires in having a magnetic tip 74, which in this example has its south pole at the distal end of the guidewire 70 and its north pole proximal to that. The magnetic element of the tip 74 could have the opposite polarity to that shown in Figure 3 in other embodiments (in which case the polarity of the driving magnets would be reversed for the reasons explained below).

Referring now to Figures 4A to 4C, these show how a guidewire of the nature shown in Figure 3 can be made to bend to significant angles with only minor movements of a guiding magnet unit. In Figure 4A a guidewire is shown having characteristics similar to those shown in Figure 3. With the guidewire 70 in unbiased condition, it tends to be straight, as shown in Figure 4A.

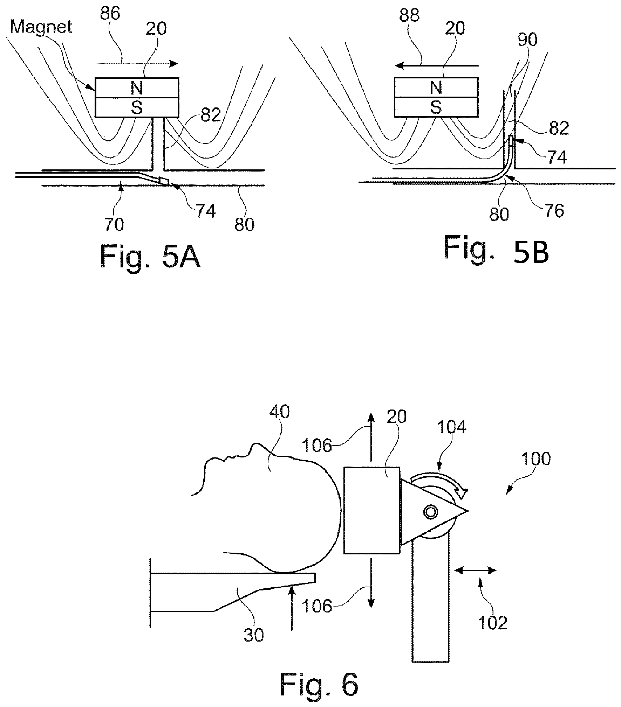

Figures 4B and 4C show how the guidewire 70 can be caused to curve under the influence of the driving magnet unit 20 having a north-to-south polarity which is opposite that of the magnet 74 at the distal end of the guidewire 70. In this example, the south pole of the driving magnet 20 faces the tip of the guidewire 70 and the south pole of the guidewire's distal magnet 74. With reference to Figure 4B, the driving magnet 20 has been positioned in magnetic range of the distal tip of the guidewire 70 and with its magnetic centre to the left (in the view of Figure 4B) of the centreline of the guidewire 70, which repels the south pole of the guidewire magnet 74 causing the distal end 76 to curve to the left also. In Figure 4B, the driving magnet 20 has been moved to the other side of the guidewire, to the right in the drawing, causing, again by repulsion, the tip of the guidewire 70 to flex in the opposite direction. The inventors have discovered that significant bending motion can be imparted to the distal end 74 of the guidewire 70 by only small adjustments in the position of the driving magnet 20 by such a magnetic repulsive arrangement. They have also discovered that this can be particularly useful in guiding a guidewire through the vasculature of a patient. An example of this can be seen in Figures 5A and 5B. In Figure 5A, a guidewire 70 is positioned within a representative vessel structure which includes a main vessel 80 and a branch vessel 82 extending, in this example, generally perpendicularly to the main vessel 80. The magnetic tip 74 of the guidewire 70 is, in Figure 5A, located substantially opposite the bifurcation to the side branch 82. The driving magnet 20 is positioned near the side branch and in Figure 5A moved, in direction of arrow 86, towards the distal end of the guidewire 70, such that the south pole of the driving magnet 20 repels the magnet tip 74 towards the opposite wall of the main vessel 80. With reference now to Figure 5B, the driving magnet 20 has been moved in the opposite direction, in what could be described as a proximal direction, that is in the direction of arrow 88, in effect exposing the magnet 74 of the tip of the guidewire 70 to the magnetic field lines 90 extending from the south to the north pole of the driving magnet 20. This causes the magnetic tip 74 to be attracted towards the opposing north pole of the driving magnet 20 and in practice into the side branch 82 of the vessel structure. Thus, by slight movement of the driving magnet 20, the distal tip of the guidewire 70, by means of its distal magnet 74, can be directed through a vessel structure in a quick and efficient manner.

The principles are used in the preferred embodiments of the magnetic driving system disclosed herein.

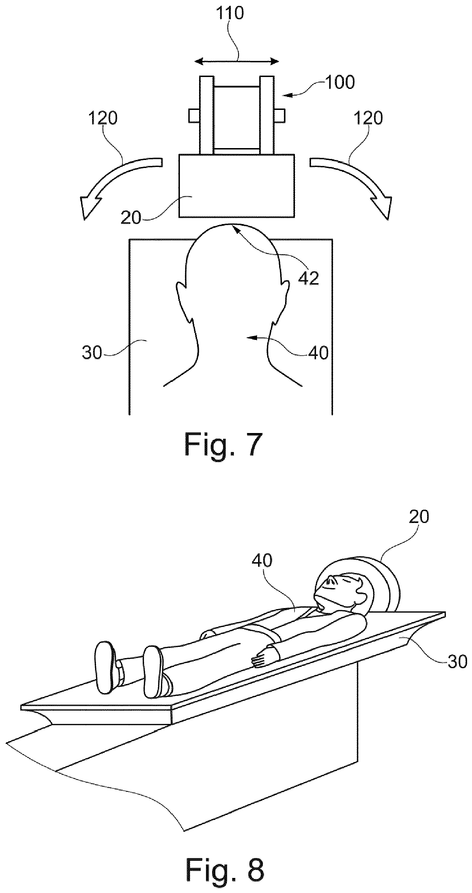

With reference first to Figures 6 and 7, these show the principles of Figures 1 A and 1 B in combination with a preferred arrangement for positioning the magnet unit 20 prior to operation thereof. With reference to Figure 6, the magnet unit 20 may be mounted on a support assembly 100 which can provide fine movement for the magnet unit 20 towards and away from the head of the patient 40, that is in direction of double arrow 102 of Figure 6, a tilting adjustment of the magnet unit 20 either way about a horizontal plane, as indicated by the arrow 104 in Figure 6, as well as upwards and downwards movement of the magnet unit 20, in the direction of arrows 106. The upward and downward movement of the magnet unit 20 could in some instances be carried out simply by tilting in the direction of the arrow 104.

With reference to Figure 7, the support assembly 100 may also provide for sideways movement of the magnet unit 20, as shown by the double arrow 110, as well as a yaw adjustment as depicted by arrows 120 in Figure 7.

The intention of these adjustments of the magnet unit 20 is to position this appropriately with respect to the crown 42 of the patient's head 40, to what one could describe as alignment with a centre point of the patient's head. This provides a useful starting location of the magnet unit 20 for driving a guidewire into the patient's neurological vessels. Typically, in such procedure, the guidewire would be inserted from a patient's neck typically through the jugular vein.

An arrangement having the characteristics of Figures 6 and 7 is shown in schematic form in the perspective view of Figure 8, which shows a patient 40 lying on a table 30 which his head adjacent the movable magnetic unit 20.

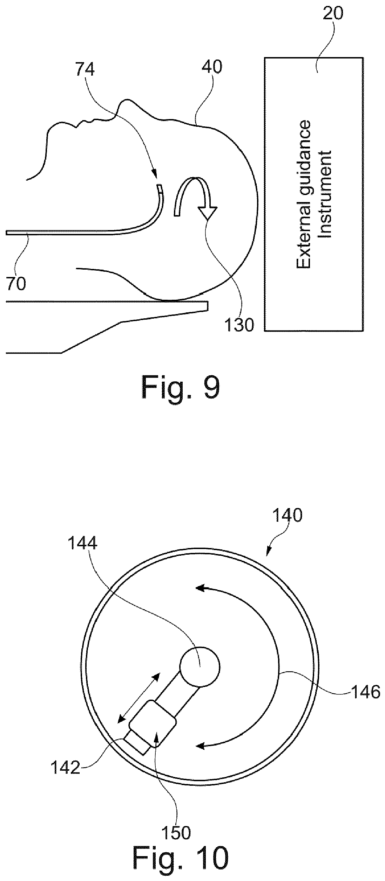

Figure 9 shows in general terms the arrangement of the apparatus of Figure 8, in which a guidewire 70 has been inserted into the cerebral vasculature of a patient 40. The tip 74 of the guidewire 70 can be curved in different directions by the magnetic unit 20 and in the preferred embodiment in a repetitive and periodic rotary motion as depicted by the arrow 130 and described in further detail below.

Figure 10 shows in schematic form a preferred magnet support 140 which includes, in this example, a support arm 142 attached to a spindle or axle 144, about which the arm 142 can rotate, as depicted by the arrow 146. The spindle or axle 144, otherwise termed a rotatable shaft, acts as the guide element. One or more magnets 150 are disposed, in this example, at one end of the arm 142, so as to rotate in either of the directions (clockwise or counter-clockwise) of arrow 146.

The magnet or magnets 150 are also, in this embodiment, able to slide along the arm 142, so as to adjust its/their position relative to the centre of rotation 144 and as a result relative to the centreline through the patient's cranium. This can adjust the relative position of the magnet(s) 150 relative to the guide wire 70. The physician can adjust the position of the magnets manually, although in other embodiments this may be done by the control system, in which case the apparatus will be provided with a drive mechanism such as a motor or solenoid for moving the magnet along the arm 142.

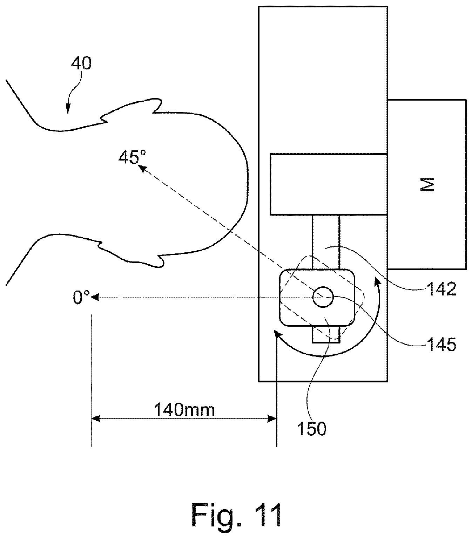

Figure 11 shows the effect produced by the rotating magnet(s) 150 of the arrangement of Figure 10 and in particular when the magnet 150 is tilted on the arm 142. For this purpose, the magnet is fitted of a pivot 145 which is in turn slidably held on the arm 142. Tilting of the magnet 150 towards the patient 40 has the effect of optimising the magnetic field within the patient's head (in this example). The distance measurement shown in Figure 11 is indicative of a typical maximum distance to cover the volume of a patient's head, which is much less that the distances required to be covered by prior art systems. The ability to move the magnet 150 along the arm 142 adds to this optimisation of magnet positioning.

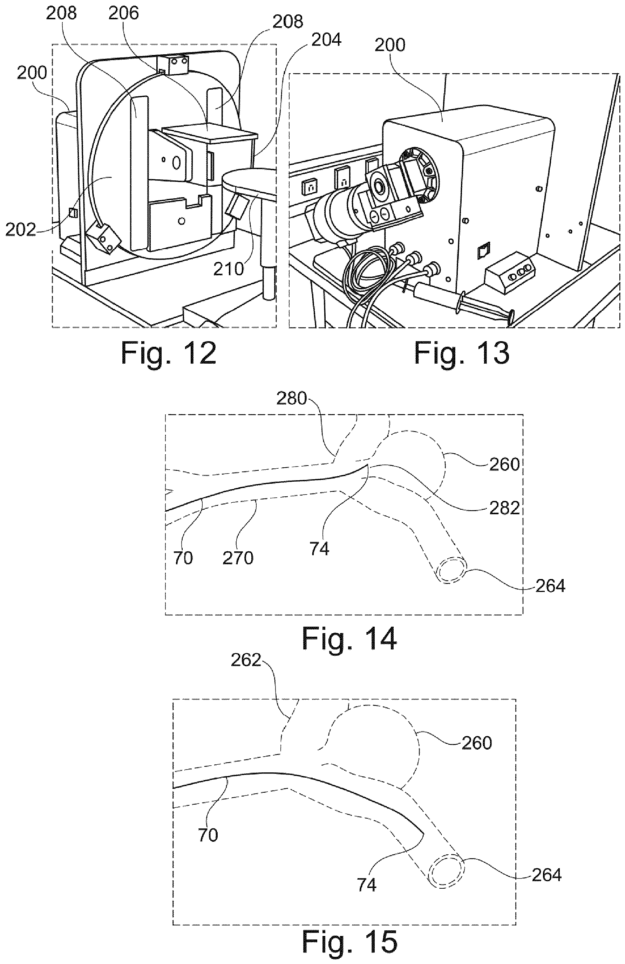

With reference now to Figures 12 and 13, these show an example of a prototype implementation of the system taught herein. The apparatus includes a drive unit 200 which houses a motor and control circuitry, as well as a rotatable drive member 202 which in this embodiment is substantially disk shaped. Attached to the rotatable drive member 202 is a magnet support assembly 204, in practice located adjacent or at the centre of the member 202, that relative to the centre of rotation of the apparatus. The magnet support 204 has, in this example, a support plate 206 onto which one or more magnets can be attached. The support plate 206 is mounted in guides 208, allowing the plate 206, and as a consequence the magnet(s) fixed thereon, to move relative to the point of rotation and relative to the patient's head when lying on support table 210.

The apparatus shown in Figures 12 and 13 uses permanent magnets but, as with all embodiments envisaged herein, could use electromagnets, in which case there will be provided an electrical supply to power the electromagnets.

The apparatus disclosed herein is, in the preferred embodiments, able to move the magnets in a repetitive periodic motion, in the case of rotational movement at a uniform rate of rotation. The rate of movement could be fixed although in some embodiments could be adjustable, for instance by the physician, in order to alter the rate and movement of the magnetic field in the zone of interest created by the magnet(s). It is to be understood that other embodiments may move the magnets linearly rather than rotationally, and/or in one or more directions of movement. The aim, in the preferred embodiments, is to create a periodically moving, for instance rotating, magnetic field which creates a repetitive pattern of movement of the end of a guide wire or other medical device deployed in a patient. The movement of the magnet(s) 20 is preferably in a single plane, which assists in providing a regular and predictable movement of the end of the medical device or tool, as well as simplifying the apparatus and optimising access to the patient.

Advantageously, the periodic motion of the magnets of the device is at a relatively low frequency in order that the distal end of the elongate medical device, such as a guidewire or catheter, will move slowly enough that a physician can manoeuvre the medical device through the vasculature of the patient in reasonable time and with reasonable efficiency. Typically, the periodic motion may be around 1 rpm or less or 1/50 or 1/60 Hertz or less for linear reciprocal motion. Movement of the magnet is preferably also relatively small. In the case of a rotating magnet arrangement, the radius of rotation may be in the region of around 70 mm to around 100 mm, but can be as low as 5 mm, in dependence on the position of the distal end of the guide wire and the tortuosity of the vasculature in which the guide wire is located. The radius can be adjusted as the guide wire is moved during the procedure. In the case of a linear motion (in one or more directions) the magnets may move within a range of 100 mm to around 200 mm. As explained above, it is not necessary to move the magnets over large distances in order to be able to direct the distal tip of the medical device as a result of the preferred arrangement disclosed herein.

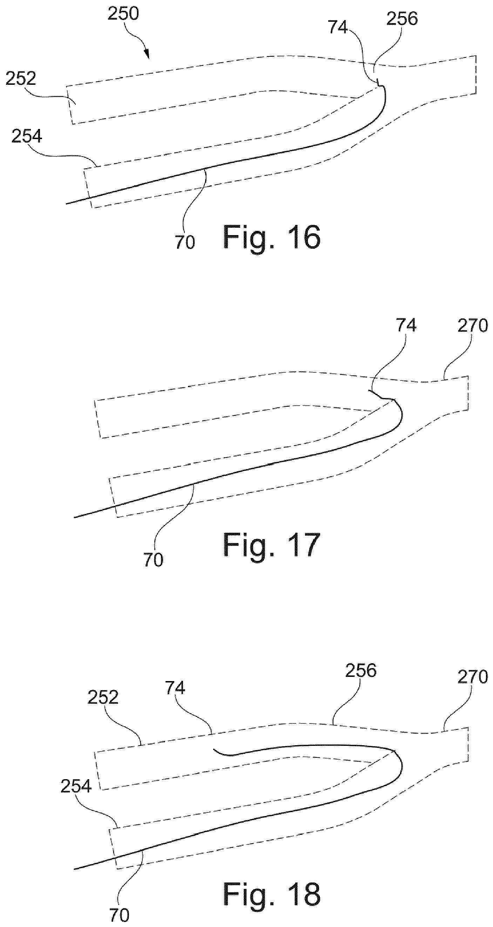

With reference now to Figures 14 to 18, these show how a guidewire 70 can be controlled through an exemplary vessel structure. The vessel structure includes a proximal end 250 (best seen in Figures 16 to 18) having two branch vessels 252, 254 connected at an acute angled bifurcation 256. The structure also includes an enlarged head section 260 with first and second branch arms 262, 264 extending from a body section 270 just proximal of the head section 260. The head section 260 has characteristics similar to those of an aneurysm occurring at a vessel T-junction.

With reference first to Figure 14, a guidewire 70 of the type disclosed above with reference to Figures 3 to 4C has been inserted through the leg vessel section 254, up through the body section 270 and is at a neck 282 of the vessel structure. The tip 74 of the guidewire 70 can be made to bend or flick around the neck portion 282 of the vessel structure by the movement of the driving magnets 20 in the manner described above. When these are moved in a periodic motion, the tip 74 will be biased periodically in different orientations, which allows the clinician to decide how to advance the guidewire 70. With reference to Figure 15, it can be seen that the guidewire 70 has been advanced into the branch section 264, achieved by waiting until the tip 74 has moved or flicked towards the branch 264 and then advancing the guidewire 70 into the branch arm. Once pushed into the branch arm, any further biasing of the tip 74 of the guidewire 70 by the moving magnets will not cause the tip 74 to move out of the branch arm, as long as it has been slid sufficiently into the branch vessel.

With reference to Figures 16 to 18, the guidewire 70 can be seen at the zone of the bifurcation 256 of the two leg sections 252, 254. In this instance, the repulsive magnetic force between the guide magnet(s) 20 and the tip 74 of the guidewire 70 is used to urge the tip 74 from one leg section 254 into the other leg section 252. This can be seen particularly in Figures 16 and 17. Once the tip 74 is aligned towards the branch leg 252, typically at the moment in time where the relative position of the guide magnet 20 is substantially opposite the location of the tip 74, the guidewire 70 can be pushed further into the leg section 254, causing the distal end of the guidewire to flex around the V-shaped junction and into the second leg section 252.

The tip 74 of the guidewire 70 can be made to follow a variety of orientations in this manner and as a result to pass through tortuous vasculature and side branches within a networks of vessels.

The inventors believe that the system disclosed herein can enable the placement of a guidewire or other medical device in the cerebral vessels of a patient within a matter of minutes, compared to the hours that it currently takes with a conventional guidewire. This is a significant advantage of current medical procedures.

In the preferred embodiments, the physician is able to move the endoluminal medical device under the influence of the periodic, and repetitive, moving magnetic field without the need for any other control input. This is considered a significant advantage in that the apparatus can be made less complex with less control inputs and can also provide a mechanism which quickly becomes intuitive and simple for the physician to implement.

The teachings herein are not limited to the provision of a guidewire as they can be used with a variety of other elongate medical devices, including probes, tools, catheters and so on. With these other embodiments, the medical device will be provided with a magnet at its distal end, similar to that disclosed above. Advantageously, the medical device would also be provided with a more flexible distal end, again in a manner similar to the guidewire depicted in Figures 4A to 4C, which will assist in the guiding of the distal end of the device through a patient's vascular system.

Although the embodiments described above are directed to apparatus suitable for guiding a medical device within the cerebral zone of a patient, the skilled person will appreciate that the system could be designed for other body parts of a patient. The principles of such other embodiments are the same as those described herein.

It is not necessary for the magnets to be placed adjacent the crown of a patient's head or even in the orientation shown. Other embodiments are envisaged, with the magnets being positioned laterally of the patient's head, and even above and below. However, the arrangement described above and depicted in the drawings is the preferred at least for the reasons given above.

The magnet(s) could, instead of moving in a rotary motion, move in a linear motion, for instance backwards and forwards, or in at least two directions, such as in two orthogonal directions or even in a greater number of directions.

The system may be entirely automated but may also allow for some fine adjustment such as described above and also during operation, for instance in terms of the speed of movement, the period of movement, distance of movement and also be temporarily stopped and started if desired. As such changes can be relatively simple, they could be controlled by a simple input such as a foot pedal, a hand dial and so on.

It will be appreciated that the magnet arrangement can be located quite close to a patient's body, particularly in light of the single plane movement of the preferred embodiments and also the relatively small movement required by the magnets in order to move a guidewire or other medical device. This can also result in a system which requires weaker magnets as the guiding magnet(s) can be located much closer to the area of interest, which has further advantages in terms of effects on the patient, imaging and size in complexity of the apparatus.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in British patent application number 1603636.0, from which this application claims priority, and in the abstract accompanying this application, are incorporated herein by reference.

## Claims

1. A medical magnetic guidance system for guiding a medical device deployed in a patient, the system including:
a patient support;
a magnet assembly disposed adjacent the patient support, the magnet assembly including a movable magnet support, and one or more magnets attached to the support; the movable magnet support being mounted on a guide element;
a drive mechanism coupled to the magnet support and configured to move the magnet support and the one or more magnets attached thereto in the guide element in a repetitive periodic motion.

2. A system according to claim 1, wherein the guide element includes a rotary coupling to magnet support, the drive mechanism being operatively connected to the rotary coupling so as to move the magnet support in a rotary motion.

3. A system according to claim 1 or 2, wherein the magnet support is mounted on a rotatable axle;
wherein the drive mechanism is coupled to the axle of the magnet support and operable to rotate the magnet support and the one or more magnets attached thereto about the axle.

4. A system according to claim 3, wherein the magnet support is rotatable in a single plane.

5. A system according to claim 1, wherein the guide element includes a linear coupling to magnet support, the drive mechanism being operatively connected to the guide element so as to move the magnet support in a reciprocating motion.

6. A system according to any preceding claim, wherein the magnet assembly includes a vibratory coupling to magnet support, the drive mechanism being operatively connected to the vibratory coupling so as to move the magnet support in a reciprocating vibratory motion.

7. A system according to any preceding claim, wherein the patient support includes a patient head zone and a patient body zone and said magnet assembly is disposed adjacent the patient head zone; and/or wherein the medical device is a neurological device.

8. A medical magnetic guidance system for guiding a medical device deployed in a patient, the system including:
a patient support;
a magnet assembly disposed adjacent the patient support, the magnet assembly including a movable magnet support and one or more magnets attached to the support, the magnet support being mounted on a guide element having a single plane;
a drive mechanism coupled to the magnet support to move the magnet support and the one or more magnets attached thereto in the guide element in said single plane.

9. A system according to any preceding claim, wherein said single plane is lateral, above or below the patient support;
wherein the patient support includes a patient head zone and a patient body zone and said single plane is at or adjacent a crown end of the patient head zone.

10. A system according to any of claims 8 to 9, wherein the medical device is a neurological device.

11. A system according to any preceding claim, wherein the patient support lies in a plane and the at least one magnet is disposed substantially perpendicular to the plane of the patient support; wherein the at least one magnet is optionally disposed at or adjacent a top of head position of the patient support; wherein the at least one magnet is optionally disposed in a plane tangential to a top of head position of the patient support.

12. A system according to any preceding claim, wherein the one or more magnets are positioned off-centre relative to the or a patient head zone of the patient support; and/or wherein the magnet assembly is positioned substantially in contact with the patient support.

13. A system according to any preceding claim, wherein the drive mechanism is operable to move the magnet support in a periodic motion of around 1 rpm or less or 1/50 Hz or less; and/or wherein the one or more magnets move within a range of 5 millimetres to 100 millimetres.

14. A system according to any preceding claim, including an elongate medical device, the medical device including a distal end with a magnetised distal tip having a given magnetic polarisation at the distal end, the one or more magnets having a front surface, which front surface has the same magnetic polarisation as that of the distal end of the medical device such that the one or more magnets exert a magnetic repulsive force on the distal end of the medical device.

15. A system according to claim 14, wherein the medical device is a guide wire or a catheter; and/or wherein the magnet assembly is adjustable in yaw or pitch.
